# EUROPÄISCHE PATENTANMELDUNG

(11) **EP 0 864 863 A2**
(43) Veröffentlichungstag der Anmeldung: **16.09.1998**
(21) Anmeldenummer: 98250087.8
(22) Anmeldetag: 09.03.1998
(51) Int. Cl.: G01N 33/00, G01N 33/48, G01N 33/53

(54) **Verwendung von Anti-Creatinin-Antikörpern oder anderen Creatinin bindenden Substanzen zur Bestimmung von Creatinin in physiologischen Flüssigkeiten und Verfahren zu ihrer Herstellung**

(30) Priorität: 13.03.1997 DE 19710943; 06.06.1997 DE 19724794
(71) Anmelder: IMTEC IMMUNDIAGNOSTIKA GMBH, 16341 Zepernick (DE)
(72) Erfinder: Schössler, Werner, Dr.rer.nat.habil., 16341 Schwanebeck (DE); Scheller, Frieder, Prof.Dr., 16341 Zepernick (DE); Hentschel, Christian, Dr., 16356 Birkholz (DE); Warsinke, Axel, Dr., 13407 Berlin (DE); Benkert, Alexander, Dipl.-Chem., 13051 Berlin (DE); Micheel, Burkhard, Prof.Dr., 10249 Berlin (DE); Scharte, Gudrun, 13189 Berlin (DE)
(74) Vertreter: Baumbach, Friedrich

(57) **Zusammenfassung**

Die Erfindung betrifft die Verwendung von Anti-Creatinin-Antikörpern oder anderen Creatinin bindenden Substanzen zur Bestimmung von Creatinin in physiologischen Flüssigkeiten und Verfahren zu ihrer Herstellung. Zur Immunisierung werden erfindungsgemäß als Creatinin-Derivate Carrier-Molekül-gekoppelte Imidazolidinderivate eingesetzt.

## Beschreibung

Die Erfindung betrifft die Verwendung von Anti-Creatinin-Antikörpern oder anderen Creatinin bindenden Substanzen zur Bestimmung von Creatinin in physiologischen Flüssigkeiten und Verfahren zu ihrer Herstellung.

Neben der Bestimmung von Glucose und Hämoglobin, ist Creatinin der am häufigsten bestimmte Parameter in der klinischen Diagnostik. Die bisherigen Bestimmungsmethoden für Creatinin sind meist umständlich oder/und werden durch andere in der Blutprobe enthaltene Substanzen empfindlich gestört.

In der klinischen Chemie stellt die Bestimmung von Creatinin eine der wichtigsten Methoden zur Überprüfung der Nierenfunktion dar. Im Gegensatz zum Harnstoff, bleibt die Creatininkonzentration weitgehend unabhängig von der Ernährungsweise. Die Creatininkonzentration im Serum (<100µM) ist jedoch im Vergleich zur Harnstoffkonzentration gering, so daß an die Sensitivität eines Bestimmungstestes hohe Anforderungen gestellt werden. Die bisherige Bestimmung von Creatinin erfolgt im Routinelabor meist über die Jaffe-Methode: In alkalischem Medium bildet Creatinin mit Pikrinsäure einen Creatinin-Pikrat-Komplex, der der Lösung eine orangene Farbe verleiht. Die Absorption der Lösung ist der Creatininkonzentration proportional.

Die kostengünstige Jaffe-Methode hat jedoch den Nachteil, daß sie relativ unspezifisch ist. Etwa 50 Substanzen, die auch als Nicht-Creatininchromogene (z.B. Acetoacetat oder Glucose) bezeichnet werden, bilden mit Pikrat ein ähnliches Reaktionsprodukt wie Creatinin (Clin. Chem., 1980, 26, 1119).
Trotz verschiedener Modifizierungen der Methode kann Creatinin nicht mit ausreichender Genauigkeit bestimmt werden, vor allem wenn Creatininkonzentrationen kleiner als 85 µM ("creatininblinder" Bereich) bestimmt werden müssen.

Neben der chemischen Methode wurden in der Vergangenheit mehrere enzymatische Methoden etabliert, die in Bezug auf Spezifität eine deutliche Verbesserung zum Jaffe-Test zeigen. Bei den meisten sind mehrere Enzymereaktionen beteiligt.
Viele enzymatische Tests zur Bestimmung von Creatinin nutzen das Enzym Creatininase, wobei das durch die Creatininasereaktion freigesetzte Creatin mit Hilfe von anderen enzymatischen Reaktionen indiziert wird. So setzt z.B. die Creatinphosphokinase Creatinin unter ATP-Verbrauch zu Creatinphosphat und ADP um. Letztlich wird der Verbrauch an NADH spektrophotometrisch bestimmt (Scand. J. Clin. Lab. Invest., 1972, suppl. 29, 126):

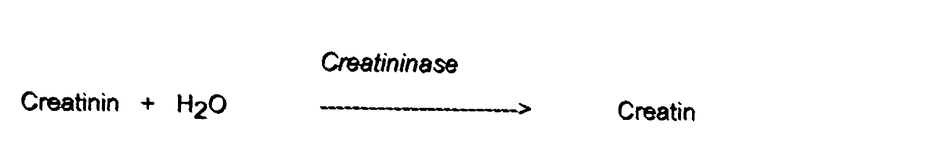

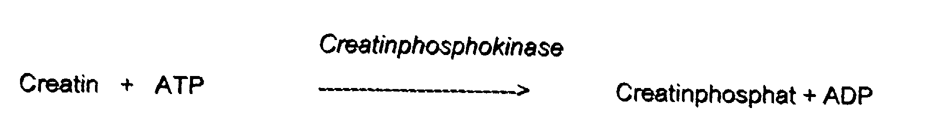

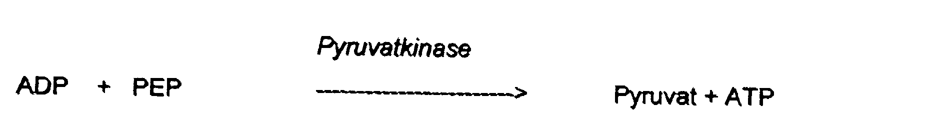

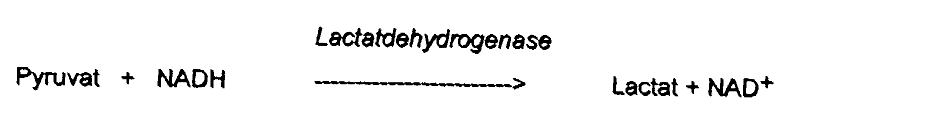

Ein anderer Test (PAP-Farbtest), nutzt die vier folgenden enzymatischen Sequenzreaktionen zur Bestimmung von Creatinin:

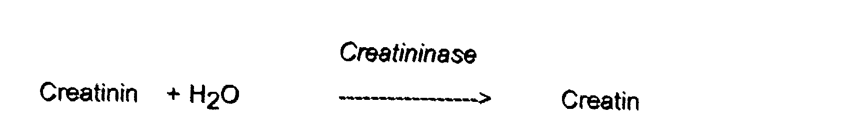

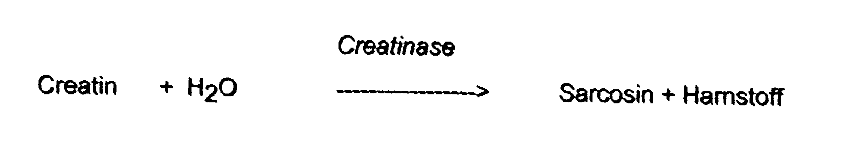

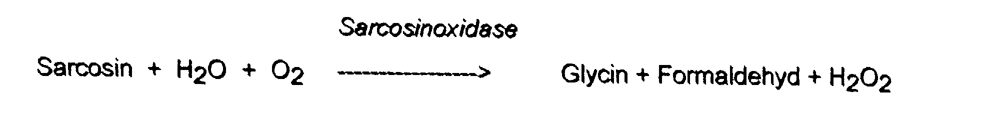

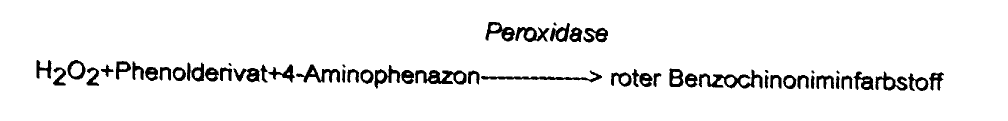

Da der Test aus mehreren enzymatischen Reaktionen besteht, können verschiedene Verbindungen (z.B. Creatin, Bilirubin, Hämoglobin, Ascorbinsäure) diesen Test stören. Am stärksten beeinflussen hohe Konzentrationen an körpereigenem Creatin die Messung, so daß sich eine Blindwertbestimmung (Test ohne Creatininase) notwendig macht oder das endogene Creatin wie im Falle des Reflotron-Teststreifens (Boehringer Mannheim) enzymatisch entfernt werden muß (Eur. J. Clin. Chem. Clin, Biochem., 1986, 31, 335).

Ein anderer enzymatischer Test, bei dem jedoch endogenes Creatin nicht die Bestimmung stört, basiert auf einer enzymatischen Umsetzung von Creatinin zu N-Methylhydantoin und Ammoniak. Ammoniak wird in einer darauffolgenden Reaktion unter NADH Verbrauch umgesetzt (Clin. Chim. Acta, 1980, 100, 21):

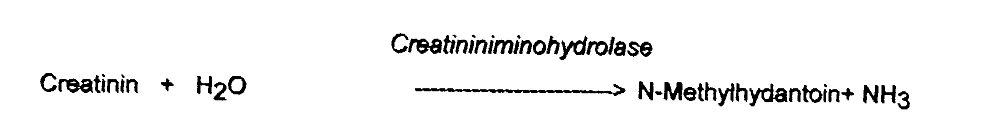

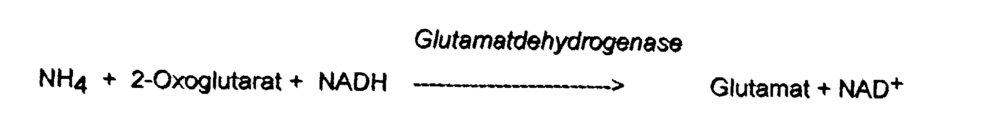

Eine Detektion des produzierten Ammoniaks kann neben der Dehydrogenasereaktion auch direkt über eine ammoniakselektive Elektrode erfolgen (Anal. Chem., 1986, 58, 145). Hohe Mengen an freiem Ammoniak im Probenmaterial schränken jedoch auch diese Methode weitgehend ein.

Beschrieben ist weiterhin eine enzymatische Methode, die ebenfalls von der Creatininiminohydrolase-Reaktion ausgeht und Anwendung im Reflotron-Teststreifen gefundet hat. Nach dieser wird nicht Ammoniak sondern das 1-Methylhydantoin weiter umgesetzt (Eur. J. Clin. Chem. Clin. Biochem., 1993, 31, 335):

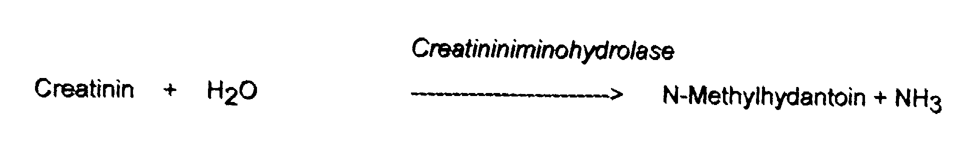

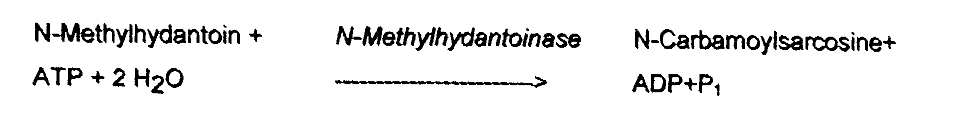

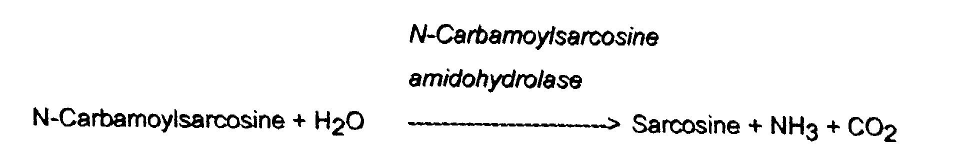

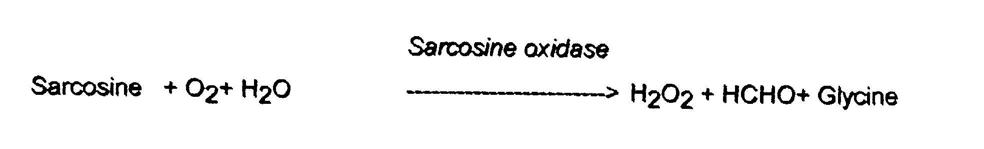

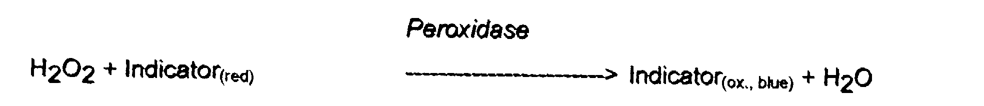

So interferieren bei diesem System zwar Creatinin und Ammoniak nicht, aber das störende Ascorbat muß aus der Probe entfernt werden. Dies geschieht mit Hilfe einer Ascorbat-Oxidase-Reaktion.

Eine Alternative stellen immunchemische Methoden dar. Immunoassays sind im Bereich der klinischen Diagnostik gut etabliert und zeichnen sich durch ihre einfache Durchführung, hohe Sensitivität und hohe Spezifität aus. Sie werden sowohl für die Bestimmung von hochmolekularen Analyten, als auch für die Bestimmung von niedermolekularen Analyten routinemäßig eingesetzt.

Einen ersten Ansatz für die immunochemische Bestimmung von Creatinin wurde 1983 von der Firma Boehringer Mannheim (DE 31 50 878 A1) beschrieben. Da Creatinin eine körpereigene Substanz ist, wurden keine Antikörper direkt gegen Creatinin entwickelt, sondern gegen N-Methylhydantoin. Im dort beschriebenen Test wird Creatinin mit dem Enzym Creatininiminohydrolase in N-Methylhydantoin umgewandelt, das dann durch die nachfolgende immunochemische Reaktion in Form einer turbidometrischen Reaktion detektiert wird.
Dieser Test hat sich aufgrund seiner komplizierten Handhabung jedoch nicht durchsetzen können. Nach wie vor gibt es zur Zeit keine einfache, automatisierbare diagnostische Methode, die sehr spezifisch und sehr empfindlich ("creatininblinder" Bereich <85 µM) Creatinin nachweisen kann.

Der Erfindung lag die Aufgabe zugrunde, eine optimale (spezifische, sensitive und präzise) Bestimmungsmethode von Creatinin sowohl zur Labor- als auch Schnelldiagnostik zu entwickeln.

Es wird allgemein davon ausgegangen, daß Creatinin als körpereigene Substanz selbst nicht immunogen ist und somit eine Antikörperbildung nicht auslösen kann. Die fehlende Immunogenität ist nicht nur der Molekülgröße des Creatinins, sondern vor allem seinem ubiquitären Vorkommen in allen Säugetieren, Vögeln und Wirbeltieren geschuldet. Creatinin ist im Blut aller Säugetiere und Vögel nachweisbar und reflektiert die Nierenfunktion des Organismus. Da sich tierisches und menschliches Creatinin in ihrer Struktur nicht unterscheiden, ist auch bei Kopplung von Creatinin als Hapten an eine hochmolekulare Trägersubstanz nicht zu erwarten, daß sich durch Immunisierung von Säugetieren oder Vögeln Antikörper gegen Creatinin induzieren lassen, da die entsprechenden Tiere gegen Creatinin immuntolerant sein sollten.

Überraschend konnten Anti-Creatinin-Antikörper und andere Creatinin bindende Substanzen bereitgestellt und zum Nachweis von Creatinin in physiologischen Flüssigkeiten eingesetzt werden. In einer bevorzugten Ausführungsvariante konnten Anti-Creatinin-Antikörper und andere Creatinin bindende Substanzen bereitgestellt werden, die keine Kreuzreaktivität bzw. Bindungsfähigkeit gegenüber Creatin oder Arginin besitzen.

Die erfindungsgemäßen Anti-Creatinin-Antikörper werden hergestellt, indem man Säugetiere, wie z.B. Mäuse, Ratten oder Kaninchen oder Vögel mit einem Carrier-Molekül gekoppeltem Imidazolidinderivat oder einem Gemisch davon, der allgemeinen Formel I immunisiert, 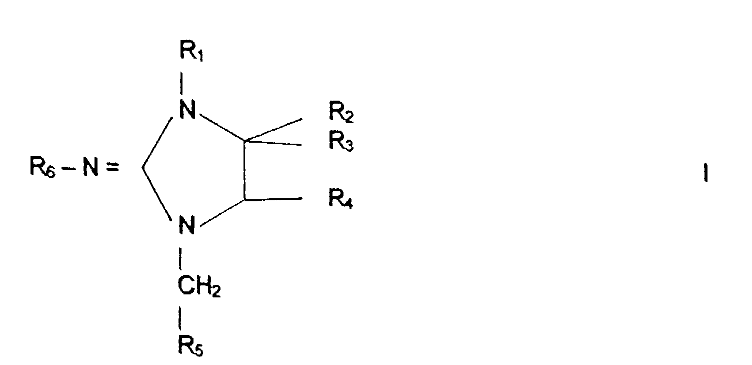 wobei
- R₁, R₄: Wasserstoff, einen Alkylrest (C₁ bis C₆), eine Alkancarbonsäure (C₁ bis C₆), ein Alkansäureamid (C₁ bis C₆), wobei der Stickstoff des Amids durch einen Alkylrest (C₁ bis C₆) substituiert sein kann, der gegebenenfalls durch N, O oder S substituiert ist,
- R₂ und R₃: Wasserstoff oder zusammen = O,
- R₅ und R₆: Wasserstoff, einen Alkylrest (C₁ bis C₆), der gegebenenfalls durch N, O oder S substituiert ist, eine Alkancarbonsäure (C₁ bis C₆), ein Alkansäureamid (C₁ bis C₆), wobei der Stickstoff des Amids durch einen Alkylrest (C₁ bis C₆) substituiert sein kann, der gegebenenfalls durch N, O oder S substituiert ist,
bedeuten,
Antiseren, die eine deutliche Reaktivität gegen Creatinin zeigen, aufreinigt, die lgG-Fraktion isoliert und die spezifischen Antikörper z.B. durch Affinitätschromatographie an immobilisiertem Creatinin oder Creatininderivat isoliert.

In einer anderen Variante werden unter Anwendung der Hybridomtechnik oder rekombinant nach an sich bekannten Verfahren mit Hilfe von Antikörperbibliotheken Antikörper gewonnen.

Bervorzugt werden Iminoimidazolidinderivate eingesetzt, besonders bevorzugte Derivate gemäß der Erfindung sind Carrier-Molekül-gekoppelte 4-(2-Imino-3-methyl-5-oxo-imidazolidin-1-yl)-buttersäure und N-(6-Amino-hexyl)-4-(2-imino-3methyl-5-oxo-imidazolidin-1-yl)-buttersäureamid.

Carrier-Moleküle im Sinne der Erfindung sind an sich bekannte hochpolymere Substanzen, wie z.B. Hämocyanin, Rinderserumalbumin, Thyroglobuline oder polymere Peptide.

Die Gewinnung von anderen Creatinin bindenden Substanzen erfolgt bevorzugt durch in vitro evolutive Verfahren aus Bibliotheken, wobei insbesondere Bibliotheken von Peptiden, Proteinen, Nukleinsäuren oder anderen organische Verbindungen benutzt werden.

Für diese Selektion werden vorteilhaft ebenfalls die zur Gewinnung von Antikörpern genutzten Verbindungen I eingesetzt.

Zur Bestimmung von Creatinin wird Analyt (Creatinin) in einer Verfahrensvariante durch gegebenenfalls auch träger-immobilisierte Anti-Creatinin-Antikörper und andere Creatinin bindenden Substanzen aus der Probe isoliert und durch ein Farbreagenz, vorzugsweise Pikrinsäure nachgewiesen. Der Vorteil gegenüber der Jaffe-Methode besteht darin, daß nur Creatinin nachgewiesen werden kann. Interferenzen werden damit vermieden.

In einer anderen Variante werden entweder der Analyt (Creatinin) oder Anti-Creatinin-Antikörper bzw. die anderen Creatinin bindenden Substanzen mit einem Marker, wie z.B. Enzymen, vorzugsweise Peroxidase, Laccase und/oder alkalische Phospatase, Fluoreszenzfarbstoffen, vorzugsweise FITC oder redoxaktiven Verbindungen, die vorzugsweise in einem Bereich von 0 mV bis -200 mV reduziert oder von 0 mV bis +200 mV oxidiert werden können, z.B. Ferrocen oder Catechol, versehen.

Der Nachweis der Bindung des Creatinins an die Antikörper oder anderen Creatinin bindenden Substanzen erfolgt nach an sich bekannten Techniken, so z.B. optisch unter Verwendung eines Chromogens, wie z.B. 4,4,4',4'-Tetramethylbenzidin, elektrochemisch unter Verwendung von Elektrodenanordnungen oder fluorimetrisch.

In bevorzugten Ausführungen der Erfindung ist ein Reaktionspartner an eine feste Phase, wie z.B eine Mikrotestplatte, einen Mikroteststreifen, ein Teststäbchen oder eine Testkugel (z.B. aus PVC oder Polystyrol) gebunden, wobei zwischen den Reaktionsschritten ein Waschprozeß stattfinden kann oder die Reaktionspartner liegen in homogener Lösung vor

So wird beispielsweise die feste Phase mit den in Lösung befindlichen Anti-Creatinin-Antikörpern oder den anderen Creatinin bindenden Substanzen in Kontakt gebracht und die Adsorption des Antikörpers oder der anderen Creatinin bindenden Substanzen erfolgt bei 1 bis 10, vorzugsweise 4 °C durch Inkubieren innerhalb von 10 bis 20, vorzugsweise 16 Stunden. Die mit den Anti-Creatinin-Antikörpem oder den anderen Creatinin bindenden Substanzen beschichtete feste Phase wird mit Körperflüssigkeiten, vorzugsweise mit Ham oder Blut, unter Verwerdung eines Markers, wie z.B. Creatinin-Enzym-Konjugat in Kontakt gebracht, gewaschen und das Creatinin wird entweder optisch, elektrochemisch oder fluorimetrisch bestimmt.

Diese Bestimmung ermöglicht mit hoher Sicherheit den Nachweis einer Niereninsuffizienz bereits im Anfangsstadium.

### Ausführungsbeispiele

### Beispiel 1

### Synthese von 4-(2-Imino-3-methyl-5-oxo-imidazolidin-1-yl)-buttersäure

80 mmol Creatinin werden in 50 ml trockenem DMF suspendiert und auf 35 °C erwärmt. Dazu addiert man 4-Brombuttersäureethylester (100 mmol) und rührt bei 80 °C ca. 2, 5 h, bis sämtliches Creatinin gelöst ist. Anschließend läßt man auf Raumtemperatur abkühlen und gibt die Reaktionslösung in 500 ml Essigester. Die entstandenen Kristalle (4-(2-Imino-3-methyl-5-oxo-imidazolidin-1-yl)-buttersäureethylester-hydrobromid) werden filtriert und gewaschen.

### Ausbeute ca. 80%

Das Hydrobromid wird anschließend zu 4-(2-Imino-3-methyl-5-oxo-imidazolidin-1yl)-buttersäure-ethylester neutralisiert.

Dazu wird 4-(2-Imino-3-methyl-5-oxo-imidazolidin-1-yl)-buttersäure-ethylesterhydrobromid (10 mmol) zu 25 ml Methanol welches 0,66 g (10 mmol) 85% iges Kaliumhydroxid enthält, gegeben und für eine Stunde bei Raumtemperatur gerührt. Im Anschluß wird das Methanol unter reduziertem Druck abdestilliert. Der Rest wird in EtOH aufgenommen und das entstandene Kaliumbromid abfiltriert .
Das Filtrat wird eingeengt und mit verdünnter NaOH (1,1 Äquivalente) 5 h unter Rückfluß verseift. Der entstandene Alkohol wird unter reduziertem Druck abdestilliert und die Reaktionslösung mit konz. HCl bis zum pH 3 versetzt. Das Lösungsmittel wird entfernt und der ölige Rückstand säulenchromatographisch gereinigt. (Kieselgel CHCl₃ / MeOH 9:1 → 6:4).

### Beispiel 2

### Synthese von N-(6-Amino-hexyl)-4-(2-imino-3-methyl-5-oxo-imidazolkiin-1-yl)buttersäureamid

40 mg 4-(2-Imino-3-methyl-5-oxo-imidazolidin-1-yl)-buttersäure (CB) werden in ca. 2 ml N-Methyl-pyrolidon (NMP) gelöst und mit je einem Äquivalent N-Hydroxybenzotriazol (HOBt) und Diisopropyl-carbodiimid (DIC) versetzt. Nach Zugabe von 1,5 Äquivalenten N-BOC-Hexamethylendiamin (Mono-Boc-HMDA) wird der Ansatz mehrere Stunden gerührt. Nach einer Mindestreaktionsdauer von drei Stunden wird stündlich eine HPLC-Analyse durchgeführt. Wenn keine Produktzunahme mehr erkennbar ist, wird der Ansatz mittels präparativer HPLC (Säule: 250*22 mm; RP 4, 300 A) aufgereinigt. Die Zielfraktion wird bis zur Trockene eingeengt und im Kolben mit ca. 1 ml Trifluoressigsäure (konz.) versetzt. Nach 30 min entfernt man die Trifluoressigsäure unter reduziertem Druck, gibt 2 ml Wasser zu und engt wiederum bis zur Trockene ein. Das Produkt wurde reanalysiert und lyophilisiert.
Die massenspektrometrische Kontrolle erfolgte mit Hilfe eines Flugzeitmassenspektrometers (MALDI-TOF).

### Beispiel 3

### Gewinnung von Antikörpern gegen Creatinin

*Immunisierungsantigen:* 20 mg (0,067 mmol) 4-(2-Imino-3-methyl-5-oxoimidazolidin-1-yl)-buttersäure (Hapten) und 20 mg Hemocyanin werden in 1 ml Aqua dest. gelöst und mit 400 µl einer 0,1 m Lösung von (1-Ethyl-3-(3dimethylaminopropyl)-carbodiimid (EDAC) in Aqua dest. versetzt, 2 h bei Raumtemperatur inkubiert und anschließend gegen dreimal 0,5 I PBS dialysiert.

*Immunisierung:* Die Primärimmunisierung von Kaninchen wird mit Hapten-Hemocyanin-Konjugat (Immunisierungsantigen), im Verhältnis 1:1 suspendiert mit komplettem Freund schen Adjuvans, durchgeführt. Das Immunogen wird generell subkutan und intramuskulär appliziert.
Die Sekundärimmunisierung erfolgt vier Wochen später nach gleichem Schema. Nach weiteren acht Wochen erhalten die Tiere eine Boosterinjektion mit gleicher Antigenmenge in inkomplettem Freund'schen Adjuvans.
Sechs Wochen nach der Primärimmunisierung erfolgt die erste Probeblutentnahme, die durch eine weitere vier Wochen später ergänzt wird. Die finale Blutentnahme wird 24 Tage nach der letzten Boosterung durchgeführt.

*Präparation der Anti-Creatinin-Antikörper*. Die Immunglobulinfraktionen der Hyperimmunseren werden durch Fällung mit Ammoniumsulfat (45% Sättigung) gewonnen.

### Beispiel 4

### Synthese des Creatinin-Enzym-Konjugates

4 mg (0,1 µmol) Meerrettich-Peroxidase (1000 U/mg; Reinheitszahl 3), gelöst in 400 µl Aqua dest. wird mit 100 µl einer 0,2 M wäßrigen Natriumperiodatlösung versetzt und 20 min bei Raumtemperatur in der Dunkelheit inkubiert. Danach werden 40 µl Ethylenglycol zugegeben und die Lösung über eine Sephadex G25-Säule (1 x 6 cm), äquilibriert in Aqua dest., entsalzt.

Die so aktivierte Peroxidase wird zu 5 mg (16,8 µmol) N-(6-Amino-hexyl)-4-(2imino-3-methyl-5-oxo-imidazolidin-1-yl)-buttersäureamid, gelöst in 500 µl 0,2 M Carbonatpuffer pH=9,5, gegeben und 2 h bei Raumtemperatur inkubiert. Zur Blockierung überschüssiger aktivierter Peroxidase wird das Reaktionsgemisch mit 100 µl einer 0,5 M Tris/HCl-Lösung pH=7,6 für 1 h bei Raumtemperatur versetzt. Anschließend erfolgt die Reduktion der Azomethinbindungen durch Zugabe von 15 µl einer 0,5 M Natriumborhydrid-Lösung in 0,5 M Carbonatpuffer pH=9,5 (6 h; 4 °C). Nach erschöpfender Dialyse gegen PBS wird dem Enzymkonjugat 15% (v/v) fötales Kälberserum und 0,01% (w/v) Thiomersal zugegeben.

### Beispiel 5

### Quantitative Bestimmung von Creatinin mittels Enzymimmunoassay mit direkter Festphasenbindung der Anti-Creatinin-Antikörper

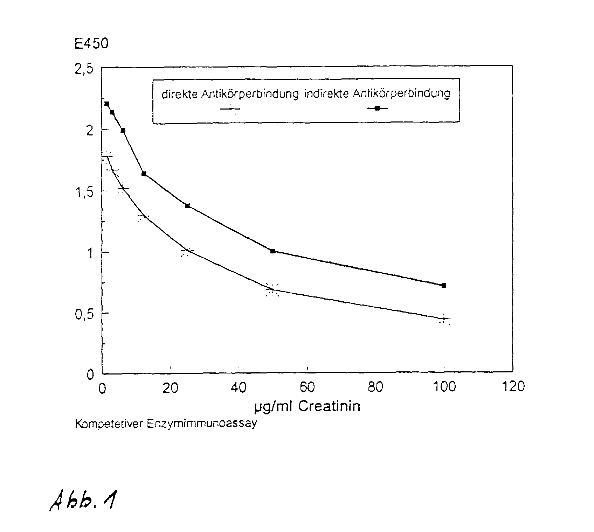

Der Analyt (Creatinin) und das Creatinin-Enzym-Konjugat konkurrieren in Lösung um die indirekt an der festen Phase insolubilisierten Anti-Creatinin-Antikörper, so daß die Menge des an die MTP gebundenen Enzyms (Peroxidase) indirekt proportional der Creatinin-Konzentration ist. Das gebundene Enzym wird durch eine Substratreaktion (Farbstoffbildung) visualisiert. Somit ist unter Mitführung von entsprechenden Standards eine Quantifizierung von Creatinin in Körperflüssigkeiten möglich (s. Abb. 1).

Die adsorptive Beladung von Polystyren-Mikrotestplatten (MTP) erfolgt mit einer Lösung von 10 µg/ml anti-Creatinin-Immunglobulin in Beladungspuffer (0,1 M Carbonatpuffer pH=9,5; 100 µl/well; 16 h; 4 °C). Danach wird die MTP dreimal mit Waschpuffer (0,05% (m/v) Tween 20 in PBS; 250 µl/well) gewaschen und 2 h bei Raumtemperatur mit Blockierungslösung (1% (m/v) Rinderserumalbumin in PBS; 150 µl/well) inkubiert und erneut dreimal gewaschen.

50 µl Serum bzw. Standard und 50 µl Creatinin-Peroxidase-Konjugat (1:50.000 in Probenpuffer (0,1% (w/v) Rinderserumalbumin; 0,05% (w/v) Tween 20; 0,01% (w/v) Thiomersal in PBS)) werden unmittelbar nacheinander auf die beladene MTP inkubiert und 30 min. bei Raumtemperatur geschüttelt. Nach dreimaligem Waschen mit 300 µl/well Waschpuffer erfolgt die Zugabe von 100 µl Substratlösung (4,4,4'4'-Tetramethylbenzidin, gebrauchsfertig). Die Farbreaktion wird nach 10 min mit 100 µl 1 n Schwefelsäure gestoppt und die Extinktionen in einem Mikrotestplatten-Reader bichromatisch bei 450 nm gegen 620 nm gemessen. Die Quantifizierung erfolgt anhand einer Standardkurve mit Hilfe einer üblichen ELISA-Software.

### Beispel 6

### Quantitative Bestimmung von Creatinin mittels Enzymimmunoassay mit indirekter Festphasenbindung der Anti-Creatinin-Antikörper

Dazu werden zunächst Antikörper gegen Kaninchen-Immunglobulin von der Ziege in einer Konzentration von 20 µg/ml in Beladungspuffer (100 µl/well; 16 h; 4 °C) an eine Polystyren-Mikrotestplatte adsorptiv gebunden. Die weitere Behandlung der MTP (Waschen, Blockieren) erfolgt wie in Beispiel 5 beschrieben. Anschließend erfolgt die Bindung der Anti-Creatinin-Antikörper durch einstündige Inkubation des Anti-Creatinin-Antiserums in einer Verdünnung von 1:25.000 in Probenpuffer (100 µl/vell).
Nach dreimaligem Waschen der Mikrotestplatte entspricht der weitere Testablauf dem in Beispiel 3 mit direkter Beladung der festen Phase.
Die Meßbereiche beider Teste (0,7 - 100 µg/ml bzw. 6,2 - 885 µmol/l) sind dabei größer als der klinisch relevante Konzentrationsbereich (s. Abb. 1).

### Beispiel 7

### Creatininmessung mit einem Biosensor auf der Grundlage von Anti-Creatinin-Antikörpern

Das BlAcore von Pharmacia ist ein vollautomatisches Microfließsystem, welches in Realzeit, ohne die Notwendigkeit einer Markierung biomolekulare Wechselwirkungen direkt analysiert. Dabei werden im wesentlichen Schichtdickenveränderungen, ausgedrückt in Resonanzeinheiten, gemessen, welche durch Anlagerung bzw. Dissoziation von wechselwirkenden Biomolekülen entstehen.

Zu diesem Zweck wurde auf einem Microchip mit aminodextranfunktionalisierter Goldoberfläche ein Analytderivat kovalent immobilisiert und die Bindung der Antikörper verfolgt.
7.1. Immobilisierung des Haptenderivates mit der Carboxylgruppe am aminofunktionalisierten Chip nach einer Standard-Carbodiimid Methode.
7.2. Vorinkubation der Creatininproben (Konzentrationen von 0,1 µg/ml bis 1 mg/ml) mit Antiserum 1:10.000 in HBS Puffer (0,01 M HEPES, pH 7,4, 0,15 M NaCl, 3 mM EDTA, 0,005 % v/v surfactant P 20) und Messung der Konzentration der überschüssigen Anti-Creatinin-Antikörper über das Assoziationsverhalten beim Durchfluß (10µl/min) des vorinkubierten Reaktionsansatzes durch die Mikrofließzelle mit dem BlAcore-Chip.
7.3. Auswertung des Anstieges der Assoziationskurven der Antikörper bei verschiedenen Creatininkonzentrationen.
7.4. Es ergab sich eine indirekte Abhängigkeit der Konzentration von Creatinin zur Signalhöhe. Je mehr freies Creatinin sich in der Lösung befand, um so mehr Antigenbindungsstellen des Antikörpers waren besetzt und um so weniger Antikörper konnten sich am immobilisierten Creatininderivat binden und ein Signal hervorrufen.
7.5. Spülen der Mikrofließzelle zwischen jeder einzelnen Messung mit Gly/HCl-Puffer pH 2.2 zur Regenerierung der Chipoberflächenbelegung. Durch Kontrollmessungen wurde sichergestellt, daß von Beginn bis zum Ende der Meßreihe die gleiche Oberflächenbelegung zur Bindung der Antikörper bereitstand.

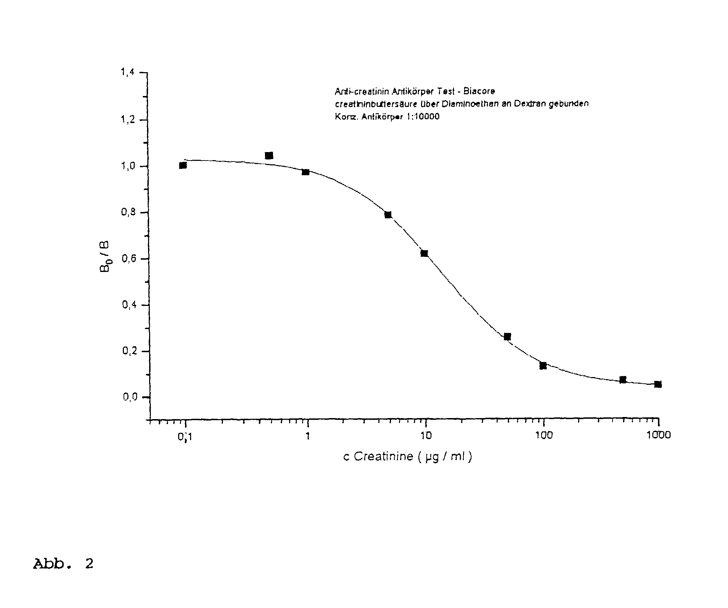

Es konnte eine Creatininabhängigkeit im Bereich von 0,1 µg/ml bis 1 mg/ml mit einem Assaymittelpunkt von ca. 14 µg/ml (122 µM) füstgestellt werden (Abb. 2). Diese Methode eignet sich zur Bestimmung von Creatinin in Realproben.

### Beispiel 8

### Gewinnung von monoklonalen Anti-Creatinin-Antikörpern mit Hilfe der Hybridomtechnik

*Immunisierung:* Als Immunisierungsantigen wurden wie für die Herstellung polyklonaler Antikörper Konjugate des Haptens 4-(2-Imino-3-methyl-5-oxoimidazolidin-1-yl)-buttersäure an Trägerproteine eingesetzt. Für die Immunisierung wurden Balb/c-Mäuse verwendet.
Die Mäuse wurden nach folgendem für verschiedene Antigene erprobtem Schema immunisiert (Micheel, B and G. Scharte, Hybridoma 12, 1993):
Erstimmunisierung durch intraperitoneale Injektion von 50 µg Hapten-Hemocyanin-Konjugat in 50 µl Phosphatgepufferter Salzlösung gemischt mit 50 µl komplettem Freund schem Adjuvans (CFA), nach 2 bis 3 Monaten Nachimmunisierung einer Maus mit der gleichen Konjugatmenge ohne Adjuvans durch intraperitoneale Injektion.
*Fusion:* Vier Tage nach der letzten Immunisierung wurden aus dem Tier Milzzellen isoliert und mit Hilfe der Hybridomtechnik zur Gewinnung von Hybridzellen eingesetzt. Die Hybridzellkolonien wurden dann auf die Anwesenheit von Anti-Creatinin-Antikörpern getestet. Die in den Immuntests positiv reagierenden Hybridzellklone wurden durch Einzelzellverdünnung kloniert, in der Zellkultur erweitert und in flüssigem Stickstoff aufbewahrt.
Die selektierten Hybridzellklone B90-AB2, B90-AD10, B90-AE3, B90-AF1, B90-AH5. B90-BD2, B90-BE11, B90-BE8, B90-CB3, B90-CC2, B90-DB10, B90-DB12, B90-DF3, B90-EA5, B90-EC12, B90-ED6, B90-FF12, B90-FF8, B90-GH12, B90-HH5, B90-JC10, B90-JF11, B90-JG7 und B90-KD2 zeigten ein gutes Wachstum und eine stabile Produktion von Antikörpern gegen Creatinin. Eine besonders starke Creatininbindung zeigten Antikörper, die von den Hybridzellklonen B90-AH5, B90-BE11, B90-CC2 und B90-JF11 produziert werden.

## Patentansprüche

1. Verwendung von Anti-Creatinin-Antikörpern oder anderen Creatinin bindenden Substanzen zur Bestimmung von Creatinin in physiologischen Flüssigkeiten.

2. Verwendung von Anti-Creatinin-Antikörpern oder anderen Creatinin bindenden Substanzen nach Anspruch 1, dadurch gekennzeichnet, daß sie keine Kreuzreaktivität bzw. Bindungsfähigkeit gegenüber Creatinin oder Arginin besitzen.

3. Verwendung nach Anspruch 1 oder 2,
dadurch gekennzeichnet, daß
entweder Creatinin oder Anti-Creatinin-Antikörper bzw. die anderen Creatinin bindenden Substanzen mit einem Marker versehen sind.

4. Verwendung nach Anspruch 3,
dadurch gekennzeichnet, daß
als Marker Enzyme, Fluoreszenzfarbstoffe oder redoxaktive Verbindungen eingesetzt werden.

5. Verwendung nach Anspruch 1 oder 2,
dadurch gekennzeichnet, daß entweder Creatinin oder Anti-Creatinin-Antikörper bzw. die anderen Creatinin bindenden Substanzen direkt sensorisch nachgewiesen werden.

6. Verwendung nach Anspruch 1,
dadurch gekennzeichnet, daß
antikörpergebundenes Creatinin durch ein Farbreagenz nachgewiesen wird.

7. Verwendung nach Anspruch 1,
dadurch gekennzeichnet, daß
Creatinin chromatographisch nachgewiesen wird.

8. Verwendung nach einem der Ansprüche 1 bis 7,
dadurch gekennzeichnet, daß
die Bindung des Creatinins an die Antikörper bzw. die anderen Creatinin bindenden Substanzen optisch, elektrochemisch oder fluorimetrisch angezeigt wird.

9. Verwendung nach einem der Ansprüche 1 bis 8,
dadurch gekennzeichnet, daß
entweder die Anti-Creatinin-Antikörper bzw. die anderen Creatinin bindenden Substanzen an eine feste Phase gebunden sind und zwischen den Reaktionsschritten ein Waschprozeß erfolgt.

10. Verwendung nach einem der Ansprüche 1 bis 8,
dadurch gekennzeichnet, daß
die Reaktionspartner in homogener Lösung vorliegen.

11. Verwendung von Anti-Creatinin-Antikörpern oder anderen Creatinin bindenden Substanzen zum Nachweis einer Niereninsuffizienz.

12. Verfahren zur Herstellung von Anti-Creatinin-Antiköpern,
dadurch gekennzeichnet, daß
polyklonale oder monoklonale Antikörper durch Immunisierung von Säugetieren und/oder Vögeln mit Creatinin-Derivaten hergestellt und dann die Antikörper isoliert werden oder über Hybridomtechnik oder rekombinant mit Hilfe von Antikörperbibliotheken gewonnen werden.

13. Verfahren zur Gewinnung von anderen Creatinin bindenden Substanzen durch in vitro evolutive Verfahren aus Bibliotheken.

14. Verfahren nach Anspruch 13,
dadurch gekennzeichnet, daß
als Bibliotheken Peptide, Proteine, Nukleinsäuren oder andere organische Verbindungen verwendet werden.

15. Verfahren nach Anspruch 12 bis 14,
dadurch gekennzeichnet, daß
zur Immunisierung und/oder zur Selektion aus Bibliotheken als Creatinin-Derivate Carrier-Molekül-gekoppelte Imidazolidinderivate der allgemeinen Formel I 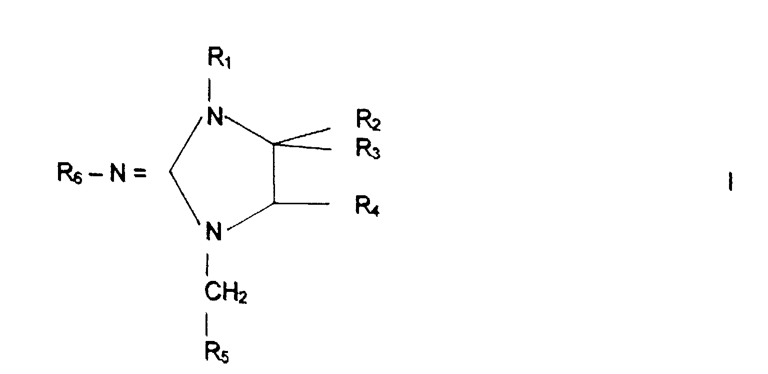 in der
R₁, R₄ Wasserstoff, einen Alkylrest (C₁ bis C₆), eine Alkancarbonsäure (C₁ bis C₆), ein Alkansäureamid (C₁ bis C₆), wobei der Stickstoff des Amids durch einen Alkylrest (C₁ bis C₆) substituiert sein kann, der gegebenenfalls durch N, O oder S substituiert ist,
R₂ und R₃ Wasserstoff oder zusammen = O,
R₅ und R₆ Wasserstoff, einen Alkylrest (C₁ bis C₆), der gegebenenfalls durch N, O oder S substituiert ist, eine Alkancarbonsäure (C₁ bis C₆), ein Alkansäureamid (C₁ bis C₆), wobei der Stickstoff des Amids durch einen Alkylrest (C₁ bis C₆) substituiert sein kann, der gegebenenfalls durch N, O oder S substituiert ist,
bedeuten, eingesetzt werden.

16. Verfahren nach Anspruch 15,
dadurch gekennzeichnet, daß
zur Immunisierung die entsprechenden Iminoimidazolidinderivate eingesetzt werden.

17. Verfahren nach Anspruch 15,
dadurch gekennzeichnet, daß Carrier-Molekül-gekoppelte
4-(2-Imino-3-methyl-5-oxo-imidazolidin-1-yl)-buttersäure und/oder N-(6-Amino-hexyl)-4-(2-imino-3-methyl-5-oxo-imidazolidin-1-yl)buttersäureamid eingesetzt werden.
